# EUROPEAN PATENT SPECIFICATION

(11) **EP 3 701 255 B1**
(45) Date of publication and mention of the grant of the patent: **11.12.2024**
(21) Application number: 17929749.4
(22) Date of filing: 26.10.2017
(51) Int. Cl.: G01N 27/26, G01N 27/404, G01N 33/00

(54) **SYSTEMS AND METHODS FOR USING A PLURALITY OF SOLID ELECTROLYTE SENSORS FOR A SELECTIVE, LOW RESOLUTION FORMALDEHYDE DETECTOR**
SYSTEME UND VERFAHREN ZUR VERWENDUNG EINER VIELZAHL VON FESTSTOFFELEKTROLYTSENSOREN FÜR EINEN SELEKTIVEN, NIEDERAUFLÖSENDEN FORMALDEHYDDETEKTOR
SYSTÈMES ET PROCÉDÉS D'UTILISATION D'UNE PLURALITÉ DE CAPTEURS À ÉLECTROLYTE SOLIDE POUR UN DÉTECTEUR DE FORMALDÉHYDE SÉLECTIF À BASSE RÉSOLUTION

(43) Date of publication of application: 02.09.2020
(73) Proprietor: Honeywell International Inc., Morris Plains, NJ 07950 (US)
(72) Inventor: XIAO, Lei, Morris Plains, New Jersey 07950 (US); PRATT, Keith Francis Edwin, Morris Plains, New Jersey 07950 (US); MU, Qinghui, Morris Plains, New Jersey 07950 (US); LIANG, Feng, Morris Plains, New Jersey 07950 (US)
(74) Representative: Haseltine Lake Kempner LLP
(86) International application number: PCT/CN2017/107755
(87) International publication number: WO 2019/080025

(56) References cited:
- WO-A1-2019/056159
- CN-A- 101 571 506
- CN-A- 101 776 640
- CN-A- 104 614 423
- CN-A- 105 987 939
- CN-A- 106 872 540
- US-A- 3 659 100
- US-A1- 2014 174 154
- US-A1- 2017 241 931

## Description

### BACKGROUND

In monitoring for the presence of various gases, other gases (e.g., carbon monoxide (CO)) can be present that can also react within the sensor. For example, an electrode of the sensor can comprise a catalyst that can catalyze the reaction of both a target gas and an interferent gas (e.g., carbon monoxide). As a result, the presence of the interferent gas may create a cross-sensitivity in the sensor, resulting in a false impression that greater levels of the target gas are present in the ambient gases than are actually present. Due to the danger presented by the presence of various target gases, the threshold level for triggering an alarm can be relatively low, and the cross-sensitivity due to the presence of the interferent may be high enough to create a false alarm for the target gas sensor.

CN101571506A describes a formaldehyde technique field, specifically, relating to a kind of low concentration formaldehyde sensor.

US2014/0174154A describes a method of selectively sensing the concentration of a target gas in polluted ambient air comprises the steps of providing a target gas sensor (220) sensitive to the target gas; providing a first gas flow derived from the ambient air, from which first flow the target gas is substantially removed.

CN104614423A describes a portable formaldehyde detecting method, in particular to a hardware anti-interference circuit based on a subtractor and a digital filtering formaldehyde detecting method, belonging to the field of detecting technology.

WO2019/056159A1 describes an electrochemical formaldehyde sensor and a method of detecting formaldehyde are disclosed. The electrochemical formaldehyde sensor may comprise a housing; an electrolyte disposed within the housing; and a plurality of electrodes in contact with the electrolyte within the housing, wherein the plurality of electrodes comprise a working electrode comprising iridium and an applied potential of between approximately 0.9 to IV relative to a reversible hydrogen electrode; and a counter electrode.

### SUMMARY

The invention is defined in the independent claims, to which reference should now be made. Advantageous features are set out in the dependent claims. In an embodiment, a method for determining the concentration of a second target gas while in the presence of a first target gas may comprise operating a first sensor under a first operating condition, wherein the first sensor is part of a sensor assembly; operating a second sensor under a second operating condition, wherein the second sensor is part of the sensor assembly, and wherein the second operating condition is different from the first operating condition; detecting at least one target gas by the first sensor; detecting at least one target gas by the second sensor; processing a signal output from the first sensor with a signal output from the second sensor; and determining a concentration of the first target gas and second target gas based on the processed output signals.

In an embodiment, a sensor assembly configured to detect a second target gas in the presence of a first target gas may comprise a first sensor comprising a first operating condition; a second sensor comprising a second operating condition, wherein the first operating condition is different from the second operating condition; and a processor configured to receive a first output signal from the first sensor; receive a second output signal from the second sensor; process the received output signals by comparing them; and determine a concentration of the first target gas and second target gas based on the processed output signals.

### BRIEF DESCRIPTION OF THE DRAWINGS

For a more complete understanding of the present disclosure, reference is now made to the following brief description, taken in connection with the accompanying drawings and detailed description, wherein like reference numerals represent like parts.
FIG. 1 illustrates a schematic diagram of a sensor according to an embodiment not forming part of the invention.
FIG. 2 illustrates a sensor assembly according to an embodiment forming part of the invention.
FIGS. 3A-3B illustrate exploded views of a first sensor and a second sensor according to an embodiment forming part of the invention.

### DETAILED DESCRIPTION

It should be understood at the outset that although illustrative implementations of one or more embodiments are illustrated below, the disclosed systems and methods may be implemented using any number of techniques, whether currently known or not yet in existence. The disclosure should in no way be limited to the illustrative implementations, drawings, and techniques illustrated below, but may be modified within the scope of the appended claims.

The following brief definition of terms shall apply throughout the application:
The term "comprising" means including but not limited to, and should be interpreted in the manner it is typically used in the patent context;
The phrases "in one embodiment," "according to one embodiment," and the like generally mean that the particular feature, structure, or characteristic following the phrase may be included in at least one embodiment of the present invention, and may be included in more than one embodiment of the present invention (importantly, such phrases do not necessarily refer to the same embodiment);
If the specification describes something as "exemplary" or an "example," it should be understood that refers to a non-exclusive example;
The terms "about" or "approximately" or the like, when used with a number, may mean that specific number, or alternatively, a range in proximity to the specific number, as understood by persons of skill in the art field; and
If the specification states a component or feature "may," "can," "could," "should," "would," "preferably," "possibly," "typically," "optionally," "for example," "often," or "might" (or other such language) be included or have a characteristic, that particular component or feature is not required to be included or to have the characteristic. Such component or feature may be optionally included in some embodiments, or it may be excluded.

Embodiments of the disclosure include systems and methods for detecting formaldehyde without a cross-sensitivity to carbon monoxide. Demand for domestic formaldehyde detectors (for personal use) may increase in areas with growth in the housing market, where people may be exposed to health damage due to exposure to low levels of formaldehyde, particularly in newly built homes (as formaldehyde exists in construction supplies and paints). Current detectors for formaldehyde typically suffer from cross-sensitivity to carbon monoxide (CO), where CO may exist in larger concentrations (when compared to the levels of formaldehyde). This cross-sensitivity may cause false alarms and prevent effective detection of formaldehyde. Also, high quality detectors currently available (that may not suffer from these cross-sensitivity issues) may be prohibitively expensive.

Embodiments of the disclosure may employ two solid electrolyte sensors (SECS) operating under two different conditions. For example, the sensors may operate with two different bias potentials and/or with two different filters. The two sensors may respond differently when exposed to formaldehyde than they do when exposed to CO (and other cross-sensitivity gases), allowing the response related to formaldehyde to be isolated and detected. The low cost of the SECS may allow for more widespread domestic use, providing users with an affordable selective formaldehyde detector. Additionally, by comparing the outputs from the two sensors, noise in the signal(s) may be cancelled out, allowing for high resolution in the detected signal. This improved resolution may allow for lower concentrations of formaldehyde to be detected.

It may be known in the art for a gas detector to use multiple sensors to provide selectivity. However, the disclosed embodiments comprise two sensors operating under different conditions, which may provide additional detection benefits, as described here. For example, a formaldehyde detector may comprise two different bias voltages applied to the two sensors. As another example, a formaldehyde detector may comprise different filters for the two sensors, which may provide increased resolution and selectivity for formaldehyde. These two examples of different operating conditions are used simultaneously in the same formaldehyde detector.

The two-sensor design can be utilized by determining a difference in the response of the two sensors. The sensor response may vary due to the difference in bias voltage, which may provide a difference in sensitivity, response time, and noise towards gases (e.g., formaldehyde, CO, and other organic gases). The differences in the response of the two sensors can be interpreted to identify two or more gases. Noise in the signals may also be canceled out to achieve selectivity and high resolution.

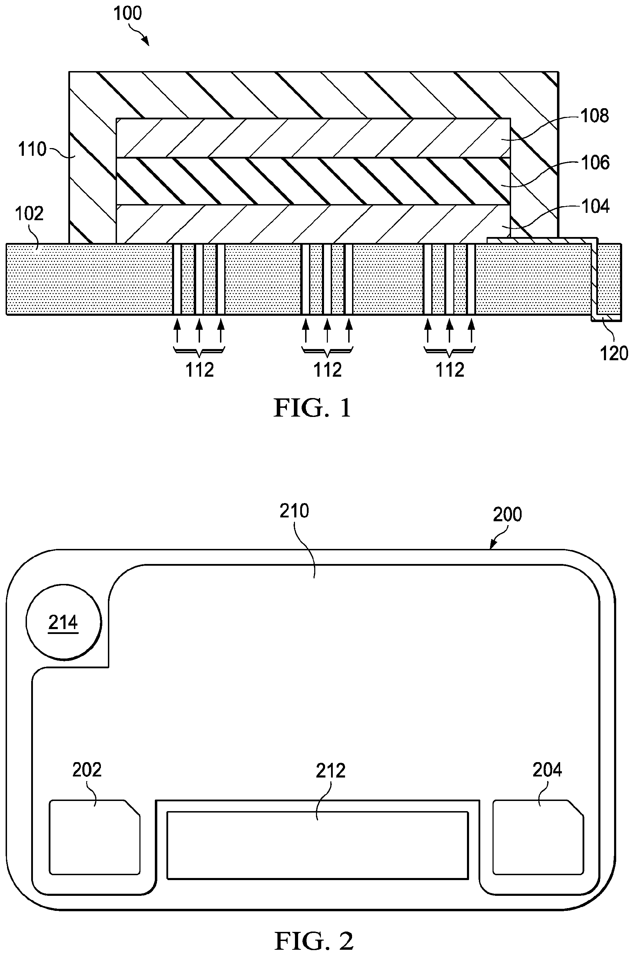

Referring now to FIG. 1, an exemplary embodiment of a sensor 100 is shown, wherein the sensor 100 may comprise a plurality of layers attached to a substrate 102. The substrate 102 may comprise an alumina ceramic material, and may comprise one or more diffusion channels 112 through the thickness of the substrate, where the diffusion channels 112 may allow gas flow into the sensor 100 from the surrounding environment. In some embodiments, the sensor 100 may comprise a first layer 104 (e.g., which may be a catalytic and/or electrode layer) configured to allow gas transfer into the sensor 100 and to the other layers of the sensor 100. In some embodiments, the first layer 104 may comprise a platinum (Pt) and ionic solution material. Although the diagram of FIG. 1 only shows one electrode layer (i.e., catalytic layer, first layer 104), the sensor 100 may comprise two or three electrodes that are co-planar with each other. In some embodiments, the first layer 104 may comprise between one and three electrodes, which may include a sensing (or working) electrode, a counter electrode, and/or a reference electrode.

In some embodiments, the sensor 100 may comprise a second layer 106 (e.g., which may be a humidification layer) configured to absorb any humidity within the sensor 100 which may prevent electrolyte from a third layer 108 from flooding the electrodes located within the first layer 104. In some embodiments, the second layer 106 may comprise silicon dioxide (SiO₂) and an ionic solution material. In some embodiments, the sensor 100 may comprise a third layer 108 (e.g., which may comprise an electrolyte layer) configured to provide electrolyte to facilitate ionic conduction between the electrodes. Optionally, the third (electrolyte) layer 108 may also be configured to provide a reservoir of water to enable the sensor to be operated over a range of humidity conditions. In some embodiments, the third layer 108 may comprise a mixture of sulfuric acid (H₂SO₄) and/or polyvinylpyrrolidone (PVPY), where the PVPY may serve to immobilize the sulfuric acid electrolyte.

In some embodiments, the sensor 100 may comprise a fourth layer 110 (e.g., which may comprise a sealing layer) configured to seal with the substrate 102 to provide an air-tight seal for the sensor 100. This sealing layer 110 may prevent air flow into the sensor 100 except for at the diffusion channels 112. In some embodiments, the sealing layer 110 may comprise a silicone material. In some embodiments, the sensor 100 may comprise one or more electrical contacts 120 that extend out of the sensor 100 to provide electrical connection(s) to other elements of a gas detector. In some embodiments, the sensor 100 may comprise up to three electrical contacts 120 for each of three electrodes that are located within the first layer 104.

Referring to FIG. 2, a sensor assembly 200 is shown where the sensor assembly 200 may comprise at least part of a formaldehyde gas detector. The sensor assembly 200 may comprise at least two sensors 202 and 204 located within the sensor assembly 200. In some embodiments, the sensors 202 and 204 may comprise electrochemical sensors. In some embodiments, the first sensor 202 may operate under a first condition and the second sensor 204 may operate under a second condition, wherein the outputs from the first sensor 202 and/or the second sensor 204 may be adjusted by adjusting the operating conditions. In some embodiments, the sensor assembly 200 may comprise a power source 212 (e.g., a battery) configured to power the elements of the sensor assembly 200. In some embodiments, the sensor assembly 200 may comprise one or more other components, such as component 214, which may comprise additional sensor elements, communication elements, electrical elements, and/or any other component 214 that may be located within the sensor assembly 200.

The different operating conditions applied to the first sensor 202 and the second sensor 204 are a difference in bias voltage and a difference in filtering conditions. By comparing the signal outputs from the two sensors 202 and 204, the gas concentrations may be predicted without the sensor(s) 202 and 204 needing to reach a steady-state. Additionally, any common mode signals (e.g., temperature, pressure, and/or humidity transients, and/or electrical interference) in the signal output from each of the sensors 202 and 204 may be cancelled out by comparing the signals.

The sensor assembly 200 may comprise a printed circuit board (PCB) 210 which may comprise one or more electrical connection elements configured to connect with the sensors 202 and 204. In some embodiments, the PCB 210 may be configured to apply one or more controls to the sensors 202 and 204 (i.e., the PCB 210 may control one or more operating conditions of the sensors 202 and 204). For example, the PCB 210 comprises one or more elements configured to apply a bias voltage to one or both of the sensors 202 and 204. The PCB 210 may comprise a processor, a memory, and other elements as would be known to those skilled in the art.

In some embodiments of the sensor assembly 200, the first sensor 202 comprises a first bias voltage while the second sensor 204 comprises a second bias voltage, where the second bias voltage is different from the first bias voltage. When the two sensors 202 and 204 are operating at different bias voltages, the relative sensitivity of the sensors to different gases may be adjusted and compared. As an example, the sensors 202 and 204 may comprise sensors configured to detect a first gas, where the sensor response to the first gas may not change with changes in bias voltage (e.g., because of diffusion limitations). However, the response of the sensors 202 and 204 to other gases, or a second gas, may be changed by adjusting the bias voltage applied to the sensors. In some embodiments, the sensitivity to other gases may be increased by increasing the bias voltage. If two different bias voltages are applied to the two different sensors 202 and 204, the signals from the two sensors 202 and 204 may be compared and/or processed to determine the response that is caused by a gas other than the first gas (i.e., the second gas). This information may be used to determine a concentration of the first and second gas. Algorithms that may be used to process the sensor outputs may consider the specific type of sensor(s), the gas flow rate(s) to the sensor(s), the effects of the bias voltage(s) applied to the sensor(s), operating conditions (e.g., temperature, pressure, humidity), among other things. Additionally, the transient behavior of the sensors 202 and 204 may be different due to the different operating conditions (i.e., different bias voltages), and therefore time dependence may be accounted for in an algorithm that processes the signals from both of the sensors 202 and 204.

As an example, the first gas may comprise CO and the second gas may comprise formaldehyde. The sensor assembly may be configured to detect both CO and formaldehyde based on the outputs of the two sensors 202 and 204. The bias voltages applied to the two sensors may be between zero (i.e., no bias voltage) and approximately 300 mV.

In some embodiments, only one sensor may have an applied bias voltage. In some embodiments, both sensors may have an applied bias voltage, where the applied bias voltage is different for the two sensors. In some embodiments, the sensor assembly 200 may comprise more than two sensors (i.e., a plurality of sensors), where each sensor of the plurality of sensors may comprise a different bias voltage, and wherein the number of gases that can be detected by the sensor assembly 200 may be equal to the number of sensors.

Each of the two (or plurality) of sensors 202 and 204 may comprise a gas channel configured to allow gas flow into the sensor(s) 202 and 204 from the external environment (e.g., similar to the diffusion channels 112 described in FIG. 1). In some embodiments, the gas channels to the sensors 202 and 204 may be separate from one another. In some embodiments, the gas channels to the sensors 202 and 204 may be configured to provide an equal gas flow to each of the sensors.

In the sensor assembly 200, the sensors 202 and 204 comprise different filtering elements configured to filter certain gases from the airflow into the sensor. For example, the first sensor 202 may comprise a first filter while the second sensor 204 may comprise a second filter, wherein the first filter is configured to filter differently than the second filter.

As an example, the sensors 202 and 204 may comprise sensors configured to detect a first gas, and the sensor assembly 200 may be configured to also detect a second gas. To accomplish detection of the second gas, the first sensor 202 may comprise a filter configured to capture/block the second gas, while the second sensor 204 does not comprise a filter. Therefore, the first sensor 202 may produce a signal in response to the first gas, while the second sensor 204 may produce a signal in response to the first gas and the second gas.

During operation, the output of the first sensor 202 (that does not include the second gas) may be compared to the output of the second sensor 204 (that does include the second gas) to determine a concentration of the second gas. In some embodiments, the signal generated by the sensor(s) 202 and 204 in response to the first gas may be significantly higher than the signal generated by the sensor(s) 202 and 204 in response to the second gas. In some embodiments, the first gas may typically be present in much higher concentrations than the second gas. As an example, the determined concentration of formaldehyde may be approximately 100 times less than the concentration of carbon monoxide. As an example, the ratio of the carbon monoxide concentration to the formaldehyde concentration may be approximately 100:1. As another example, the ratio of the carbon monoxide concentration to the formaldehyde concentration may be approximately 500:1. In some embodiments, it may be more difficult to filter the first gas from entering the sensor(s) 202 and 204 than to filter the second gas.

Algorithms that may be used to process the sensor outputs may consider the specific type of sensor(s), the gas flow rate(s) to the sensor(s), the effects of the bias voltage(s) applied to the sensor(s), operating conditions (e.g., temperature, pressure, humidity), among other things. Testing may be completed on a prototype sensor assembly to determine the preferred or optimum algorithm processing. Additionally, the transient behavior of the sensors 202 and 204 may be different due to the different operating conditions (i.e., different filtering conditions), and therefore time dependence may be accounted for in an algorithm that processes the signals from both of the sensors 202 and 204. For example, the gas flow into the first sensor 202 may be slower than the gas flow into the second sensor 204 when the first sensor 202 comprises a filter and the second sensor 204 does not.

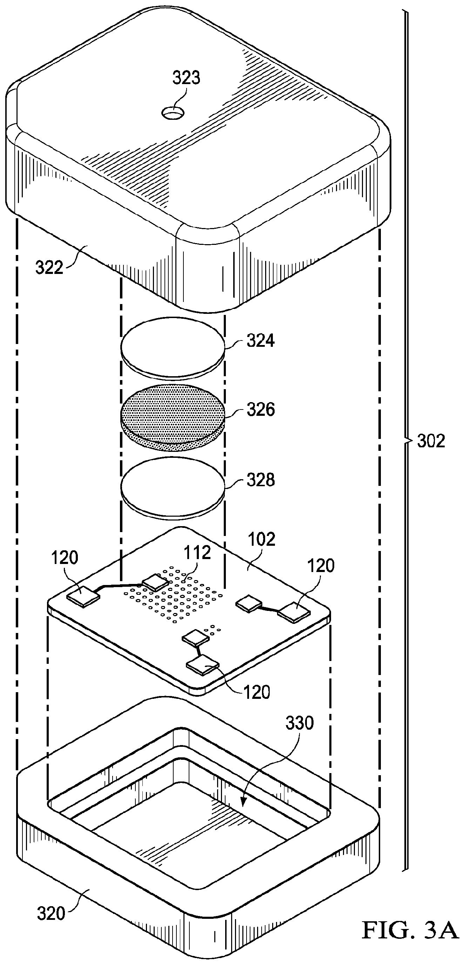

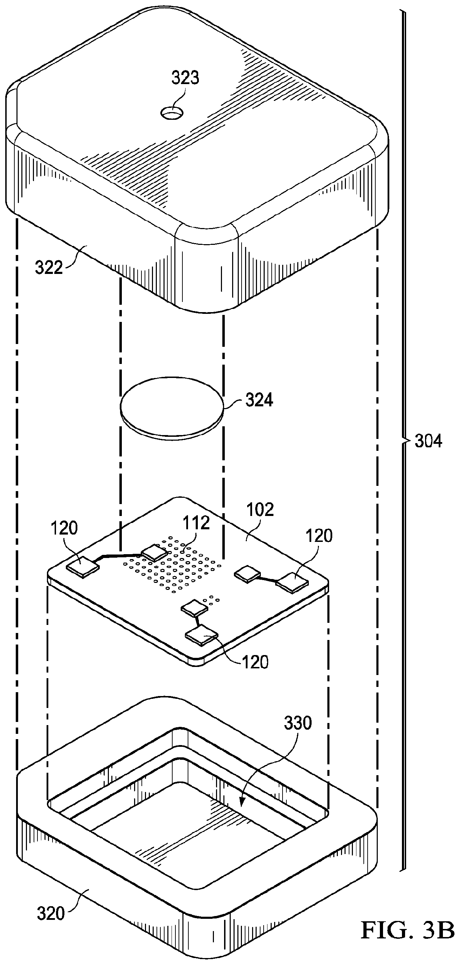

As an example, FIGS. 3A-3B illustrate a first sensor 302 and a second sensor 304 comprising different filtering conditions (where the sensors 302 and 304 may be similar to the sensors 202 and 204). The first sensor 302 and the second sensor 304 may comprise a bottom housing 320 and a top housing 322, where the top housing 322 comprises an air inlet 323. The first sensor 302 and the second sensor 304 may also comprise a substrate 102 (e.g., as described in FIG. 1), as well as one or more diffusion channels 112 in the substrate 102 and one or more electrical contacts 120. The bottom housing 320 may comprise a cavity 330 configured to hold the substrate 102 (and other layers described in FIG. 1). In some embodiments, the first sensor 302 and/or the second sensor 304 may comprise a dust filter 324 configured to prevent particulate matter from entering through the air inlet 323.

Additionally, the first sensor 302 comprises a filter 326 (which may be configured to filter the second (target) gas) located in the airflow pathway from the air inlet 323 to the diffusion channels 112. The filter 326 may comprise one or more materials configured to filter the second gas (as described above). As an example, the filter 326 may comprise a potassium permanganate impregnated glass fiber sheet. In some embodiments, the first sensor 302 may also comprise a membrane 328, configured to hold the filter 326 in place so that it does not move around within the sensor housing 320 and 322.

Some embodiments of the invention comprise a method for detecting a second target gas in the presence of a first target gas. A method may comprise detecting at least one target gas by a first sensor of a sensor assembly, where the first sensor may comprise a first operating condition. The method may comprise detecting at least one target gas by a second sensor, where the second sensor comprises a second operating condition, and the second operating condition is different than the first operating condition. The method may comprise comparing and/or processing the output signal from the first sensor and the second sensor to determine a concentration of at least one target gas. In some embodiments, the method may comprise determining a concentration of a first target gas and determining a concentration of a second target gas. In some embodiments, the method may comprise determining the concentration of the second target gas while in the presence of the first target gas. In some embodiments, the first target gas may comprise CO, while the second target gas may comprise a volatile organic compound (VOC). In some embodiments, the second target gas may comprise formaldehyde.

While various embodiments in accordance with the principles disclosed herein have been shown and described above, modifications thereof may be made by one skilled in the art without departing from the scope defined by the independent claims. The embodiments described herein are representative only and are not intended to be limiting. Many variations, combinations, and modifications are possible and are within the scope of the claims.

Accordingly, the scope of protection is not limited by the description set out above, but is defined by the claims which follow that scope including all equivalents of the subject matter of the claims. Each and every claim is incorporated as further disclosure into the specification and the claims are embodiment(s) of the present invention(s). Furthermore, any advantages and features described above may relate to specific embodiments, but shall not limit the application of such issued claims to processes and structures accomplishing any or all of the above advantages or having any or all of the above features.

Use of broader terms such as "comprises," "includes," and "having" should be understood to provide support for narrower terms such as "consisting of," "consisting essentially of," and "comprised substantially of." Use of the terms "optionally," "may," "might," "possibly," and the like with respect to any element of an embodiment means that the element is not required, or alternatively, the element is required, both alternatives being within the scope of the embodiment(s). Also, references to examples are merely provided for illustrative purposes, and are not intended to be exclusive.

While several embodiments have been provided in the present disclosure, it should be understood that the disclosed systems and methods may be embodied in many other specific forms without departing from the scope as defined by the independent claims. The present examples are to be considered as illustrative and not restrictive, and the intention is not to be limited to the details given herein. For example, the various elements or components may be combined or integrated in another system or certain features may be omitted or not implemented.

Also, techniques, systems, subsystems, and methods described and illustrated in the various embodiments as discrete or separate may be combined or integrated with other systems, modules, techniques, or methods without departing from the scope of the present disclosure. Other items shown or discussed as directly coupled or communicating with each other may be indirectly coupled or communicating through some interface, device, or intermediate component, whether electrically, mechanically, or otherwise.

## Claims

1. A method for determining the concentration of a second target gas while in the presence of a first target gas, the method comprising:
operating a first sensor (202) under a first operating condition, wherein the first sensor is part of a sensor assembly (200);
operating a second sensor (204) under a second operating condition, wherein the second sensor is part of the sensor assembly (200); and wherein:
the second operating condition is different from the first operating condition, and
the first operating condition comprises a first bias voltage and the second operating condition comprises a second bias voltage, the second bias voltage being different from the first bias voltage, wherein the first sensor comprises a first filter and the second sensor comprises a second filter different from the first filter, and wherein the first sensor comprises a membrane (328) configured to hold the first filter;
detecting at least one target gas by the first sensor;
detecting at least one target gas by the second sensor;
processing a signal output from the first sensor with a signal output from the second sensor; and
determining a concentration of the first target gas and determining a concentration of the second target gas based on the processed output signals.

2. The method of claim 1, wherein the first target gas comprises carbon monoxide.

3. The method of claim 1, wherein the second target gas comprises a volatile organic compound.

4. The method of claim 1, wherein the second target gas comprises formaldehyde.

5. The method of claim 1, wherein one of the first bias voltage and the second bias voltage comprises a zero bias voltage.

6. The method of claim 1, wherein one of the first bias voltage and the second bias voltage comprises approximately 300 mV.

7. The method of claim 1, wherein one of the first bias voltage and the second bias voltage comprises a bias voltage between approximately 0 and approximately 500 mV.

8. The method of claim 1, wherein the first operating condition comprises a first filtering condition and the second operating condition comprises a second filtering condition.

9. A sensor assembly (200) configured to detect a second target gas in the presence of a first target gas, the sensor assembly comprising:
a first sensor (202) comprising a first operating condition;
a second sensor (204) comprising a second operating condition,
wherein the first sensor (202) comprises a first filter and the second sensor (204) comprises a second filter different from the first filter;
a processor (210) configured to:
receive a first output signal from the first sensor (202);
receive a second output signal from the second sensor (204);
**characterised in that** the processor (210) is further configured to:
process the received output signals by comparing them; and
determine a concentration of the first target gas and determine a concentration of the second target gas based on the processed output signals and the first operating condition comprises a first bias voltage and the second operating condition comprises a second bias voltage, the second bias voltage being different from the first bias voltage, and wherein the first sensor (202) comprises a membrane (328) configured to hold the first filter.

10. The sensor assembly (200) of claim 9, wherein the processor (210) is configured to determine the difference between the two output signals.

11. The sensor assembly (200) of claim 9, wherein the first operating condition allows the first sensor (202) to detect the first target gas, and wherein the second operating condition allows the second sensor (204) to detect the first target gas in combination with the second target gas.

## Patentansprüche

1. Verfahren zum Bestimmen der Konzentration eines zweiten Zielgases in der Gegenwart eines ersten Zielgases, das Verfahren umfassend:
Betreiben eines ersten Sensors (202) unter einer ersten Betriebsbedingung, wobei der erste Sensor Teil einer Sensoranordnung (200) ist;
Betreiben eines zweiten Sensors (204) unter einer zweiten Betriebsbedingung, wobei der zweite Sensor Teil der Sensoranordnung (200) ist; und wobei
die zweite Betriebsbedingung von der ersten Betriebsbedingung verschieden ist, und
die erste Betriebsbedingung eine erste Vorspannung umfasst und die zweite Betriebsbedingung eine zweite Vorspannung umfasst, wobei die zweite Vorspannung von der ersten Vorspannung verschieden ist, wobei der erste Sensor einen ersten Filter umfasst und der zweite Sensor einen zweiten Filter umfasst, der von dem ersten Filter verschieden ist, und wobei der erste Sensor eine Membran (328) umfasst, die dazu konfiguriert ist, den ersten Filter zu halten;
Detektieren von mindestens einem Zielgas durch den ersten Sensor;
Detektieren von mindestens einem Zielgas durch den zweiten Sensor;
Verarbeiten einer Signalausgabe aus dem ersten Sensor mit einer Signalausgabe aus dem zweiten Sensor; und
Bestimmen einer Konzentration des ersten Zielgases und Bestimmen einer Konzentration des zweiten Zielgases auf Basis der verarbeiteten Ausgabesignale.

2. Verfahren nach Anspruch 1, wobei das erste Zielgas Kohlenstoffmonoxid umfasst.

3. Verfahren nach Anspruch 1, wobei das zweite Zielgas eine flüchtige organische Verbindung umfasst.

4. Verfahren nach Anspruch 1, wobei das zweite Zielgas Formaldehyd umfasst.

5. Verfahren nach Anspruch 1, wobei eine aus der ersten Vorspannung und der zweiten Vorspannung eine Null-Vorspannung umfasst.

6. Verfahren nach Anspruch 1, wobei eine aus der ersten Vorspannung und der zweiten Vorspannung etwa 300 mV umfasst.

7. Verfahren nach Anspruch 1, wobei eine aus der ersten Vorspannung und der zweiten Vorspannung eine Vorspannung von etwa 0 und etwa 500 mV umfasst.

8. Verfahren nach Anspruch 1, wobei die erste Betriebsbedingung eine erste Filterungsbedingung umfasst und die zweite Betriebsbedingung eine zweite Filterungsbedingung umfasst.

9. Sensoranordnung (200), die dazu konfiguriert ist, ein zweites Zielgas in der Gegenwart eines ersten Zielgases zu detektieren, die Sensoranordnung umfassend:
einen ersten Sensor (202), der eine erste Betriebsbedingung umfasst;
einen zweiten Sensor (204), der eine zweite Betriebsbedingung umfasst,
wobei der erste Sensor (202) einen ersten Filter umfasst und der zweite Sensor (204) einen zweiten Filter umfasst, der von dem ersten Filter verschieden ist; und einen Prozessor (210), der dazu konfiguriert ist:
ein erstes Ausgabesignal von dem ersten Sensor (202) zu empfangen;
ein zweites Ausgabesignal von dem zweiten Sensor (204) zu empfangen;
**gekennzeichnet dadurch, dass** der Prozessor (210) ferner dazu konfiguriert ist:
die empfangenen Ausgabesignale zu verarbeiten, indem er sie vergleicht; und
auf Basis der verarbeiteten Ausgabesignale eine Konzentration des ersten Zielgases zu bestimmen und eine Konzentration des zweiten Zielgases zu bestimmen, und wobei die erste Betriebsbedingung eine erste Vorspannung umfasst und die zweite Betriebsbedingung eine zweite Vorspannung umfasst, wobei die zweite Vorspannung von der ersten Vorspannung verschieden ist, und wobei der erste Sensor (202) eine Membran (328) umfasst, die dazu konfiguriert ist, den ersten Filter zu halten.

10. Sensoranordnung (200) nach Anspruch 9, wobei der Prozessor (210) dazu konfiguriert ist, die Differenz zwischen den zwei Ausgabesignalen zu bestimmen.

11. Sensoranordnung (200) nach Anspruch 9, wobei die erste Betriebsbedingung dem ersten Sensor (202) ermöglicht, das erste Zielgas zu detektieren, und wobei die zweite Betriebsbedingung dem zweiten Sensor (204) ermöglicht, das erste Zielgas in Kombination mit dem zweiten Zielgas zu detektieren.

## Revendications

1. Procédé pour déterminer la concentration d'un deuxième gaz cible en la présence d'un premier gaz cible, le procédé comprenant :
le fonctionnement d'un premier capteur (202) dans une première condition de fonctionnement, dans lequel le premier capteur fait partie d'un ensemble capteur (200) ;
le fonctionnement d'un deuxième capteur (204) dans une deuxième condition de fonctionnement, dans lequel le deuxième capteur fait partie d'un ensemble capteur (200) ; et dans lequel :
la deuxième condition de fonctionnement est différente de la première condition de fonctionnement, et
la première condition de fonctionnement comprend une première tension de polarisation et la deuxième condition de fonctionnement comprend une deuxième tension de polarisation, la deuxième tension de polarisation étant différente de la première tension de polarisation, dans lequel le premier capteur comprend un premier filtre et le deuxième capteur comprend un deuxième filtre différent du premier filtre, et dans lequel le premier capteur comprend une membrane (328) configurée pour maintenir le premier filtre ;
la détection d'au moins un gaz cible par le premier capteur ;
la détection d'au moins un gaz cible par le deuxième capteur ;
le traitement d'un signal produit en sortie par le premier capteur avec un signal produit en sortie par le deuxième capteur ; et
la détermination d'une concentration du premier gaz cible et la détermination d'une concentration du deuxième gaz cible sur la base des signaux produits en sortie traités.

2. Procédé selon la revendication 1, dans lequel le premier gaz cible comprend du monoxyde de carbone.

3. Procédé selon la revendication 1, dans lequel le deuxième gaz cible comprend un composé organique volatil.

4. Procédé selon la revendication 1, dans lequel le deuxième gaz cible comprend du formaldéhyde.

5. Procédé selon la revendication 1, dans lequel l'une de la première tension de polarisation et de la deuxième tension de polarisation comprend une tension de polarisation nulle.

6. Procédé selon la revendication 1, dans lequel l'une de la première tension de polarisation et de la deuxième tension de polarisation comprend environ 300 mV.

7. Procédé selon la revendication 1, dans lequel l'une de la première tension de polarisation et de la deuxième tension de polarisation comprend une tension de polarisation entre environ 0 et environ 500 mV.

8. Procédé selon la revendication 1, dans lequel la première condition de fonctionnement comprend une première condition de filtrage et la deuxième condition de fonctionnement comprend une deuxième condition de filtrage.

9. Ensemble capteur (200) configuré pour détecter un deuxième gaz cible en la présence d'un premier gaz cible, l'ensemble capteur comprenant :
un premier capteur (202) comprenant une première condition de fonctionnement ;
un deuxième capteur (204) comprenant une deuxième condition de fonctionnement,
dans lequel le premier capteur (202) comprend un premier filtre et le deuxième capteur (204) comprend un deuxième filtre différent du premier filtre ; et
un processeur (210) configuré pour :
recevoir un premier signal produit en sortie à partir du premier capteur (202) ;
recevoir un deuxième signal produit en sortie à partir du deuxième capteur (204) ;
**caractérisé en ce que** le processeur (210) est en outre configuré pour :
traiter les signaux produits en sortie reçus en les comparant ; et
déterminer une concentration du premier gaz cible et déterminer une concentration du deuxième gaz cible sur la base des signaux de sortie traités et la première condition de fonctionnement comprend une première tension de polarisation et la deuxième condition de fonctionnement comprend une deuxième tension de polarisation, la deuxième tension de polarisation étant différente de la première tension de polarisation, et dans lequel le premier capteur (202) comprend une membrane (328) configurée pour maintenir le premier filtre,

10. Ensemble capteur (200) selon la revendication 9, dans lequel le processeur (210) est configuré pour déterminer la différence entre les deux signaux de sortie.

11. Ensemble capteur (200) selon la revendication 9, dans lequel la première condition de fonctionnement permet au premier capteur (202) de détecter le premier gaz cible, et dans lequel la deuxième condition de fonctionnement permet au deuxième capteur (204) de détecter le premier gaz cible en combinaison avec le deuxième gaz cible,
